(19) Europäisches Patentamt
European Patent Office
Office européen des brevets

(11) EP 3 016 926 B1

(12) # EUROPEAN PATENT SPECIFICATION

(45) Date of publication and mention of the grant of the patent:
**31.05.2017 Bulletin 2017/22**

(21) Application number: **14734507.8**

(22) Date of filing: **03.07.2014**

(51) Int Cl.:
***C07C 45/45*** *(2006.01)*

(86) International application number:
**PCT/EP2014/064211**

(87) International publication number:
**WO 2015/001032 (08.01.2015 Gazette 2015/01)**

(54) **METHOD OF ACYLATING AN AROMATIC COMPOUND**

VERFAHREN ZUR ACYLIERUNG EINER AROMATISCHEN VERBINDUNG

PROCÉDÉ D'ACYLATION D'UN COMPOSÉ AROMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.07.2013 EP 13175354**

(43) Date of publication of application:
**11.05.2016 Bulletin 2016/19**

(73) Proprietor: **DSM IP Assets B.V.**
**6411 TE Heerlen (NL)**

(72) Inventors:
- **HÖLDERICH, Wolfgang**
  **CH-4002 Basel (CH)**
- **EISENACHER, Matthias**
  **CH-4002 Basel (CH)**
- **AREND, Matthias**
  **CH-4002 Basel (CH)**
- **VENSCHOTT, Moritz**
  **CH-4002 Basel (CH)**

(74) Representative: **Dux, Roland**
**DSM Nutritional Products Ltd**
**Patent Department**
**Wurmisweg 576**
**4303 Kaiseraugst (CH)**

(56) References cited:
- **YADAV AND M S M MUJEEBUR RAHUMAN G D: "cation-Exchange Resin-Catalysed Acylations and Esterifications in Fine Chemical and Perfumery Industries", ORGANIC PROCESS RESEARCH AND DEVELOPMENT, AMERICAN CHEMICAL SOCIETY, US, vol. 6, 1 January 2002 (2002-01-01), pages 706-713, XP002716520, ISSN: 1083-6160, DOI: 10.1021/OP0255229 [retrieved on 2002-06-11]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 016 926 B1

Printed by Jouve, 75001 PARIS (FR)

## Description

### Technical Field

The present invention relates to the acylation of aromatic compounds.

### Background of the invention

Aromatic compounds are acylated by Friedel-Crafts reactions usually in the presence of Lewis acids, typically $AlCl_3$, $BF_3$ or $ZnCl_2$ used as homogeneous catalysts. However, these catalysts are highly toxic and corrosive. Furthermore, high amounts of catalysts are used and it is necessary to separate the catalyst after the reaction is finished completely out of the reaction mixture, which is rather difficult. Furthermore, in the working up procedure a high amount of salts due to neutralization is produced, which has to be disposed in a costly and difficult manner. The catalyst cannot be regenerated and needs to be destroyed causing also the formation of waste water.

To overcome these problems it has been proposed to use heterogeneous catalysts. US 5,817,878 proposes to use specific zeolites. However, the proposed zeolites are very expensive and the reaction needs high temperatures which increases the energy costs for the reaction. These catalysts tend to have a short service time due to coke formation and need to be regenerated in short intervals at very high temperatures, typically at temperatures above 550°C.

G.D. Yadav discloses in Org. Proc. Res. Dev. 6, 706-713 (2002) the acylation of anisole with acetic anhydride in the presence of Amberlyst 15 or Amberlyst 36, which are macroreticular sulfonic acid ion exchange resins.

However, it has been shown that these types of macroreticular sulfonic acid ion exchange resins are deactivated very fast and that their efficiency described by conversion and selectivity drop very fast during the reaction. This is very disadvantageous for their use in an industrial application as the resins need to be changed at short intervals leading to disruption of the production and increased costs for the production of acylated aromatic compounds. It has been observed, furthermore, that these catalysts have a tendency to have short catalyst service time. The short service time leads to the fact that such catalysts need to be exchanged frequently leading to additional costs and to undesired interruption of the production of acylated aromatic compounds. As a consequence the space-time-yield (STY) can be poor.

### Summary of the invention

Therefore, the problem to be solved by the present invention is to increase the catalysts service time and to disclose an energy efficient process which operates at moderate temperatures.

Surprisingly, it has been found that the method of claim 1 is able to solve all these problems.

It has been shown that specific macroreticular sulfonic acid ion exchange resins can be used for reaching remarkably longer catalyst service times without offering the problem that these catalysts are deactivated very fast. This allows constant production leading to high space-time-yield (STY).

Furthermore, it has been found that this method is very advantageous in the meanings that no solvent is needed to operate the present process. In addition to that, no complicated separation of the catalyst has to be carried out. It has been further found that it is particular advantageous in that the acylation is predominately, eventually after an intermediate isomerization step, ending up in the para-position to the alkyl or alkoxy substituents being present in the aromatic compound which are to be acylated.

Particularly preferred embodiments are subject of dependent claims.

### Detailed description of the invention

The present invention relates to a method of acylating an aromatic compound of formula (I-A) or (I-B) characterized in that the aromatic compound of formula (I-A) or (I-B) is reacted with an acylating agent of formula (II-A) or (II-B) in the presence of a macroreticular sulfonic acid ion exchange resin having a water-to-phenol shrinkage of between 25 % and 40 %

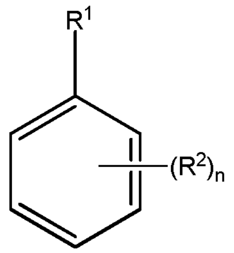
(I-A)

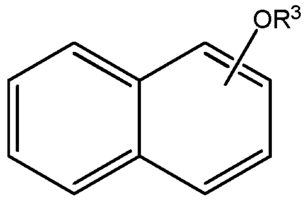
(I-B)

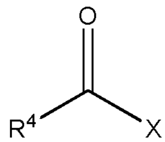
(II-A)

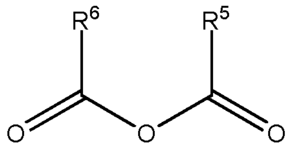
(II-B)

wherein n = 0 to 3, preferably n = 0 or 1;
$R^1$ stands for a $C_{1-4}$-alkyl group or an $OR^3$ group;
$R^2$ stands for H or for a $C_{1-8}$-alkyl group or a $C_{6-9}$-cycloalkyl group;
$R^3$ stands for $C_{1-8}$-alkyl group;
$R^4$ stands for a saturated or unsaturated $C_{1-12}$-alkyl group, a saturated or unsaturated $C_{5-12}$-cycloalkyl group or an aryl group;
$R^5$ and $R^6$ stand independently from each other for a saturated or unsaturated $C_{1-12}$-alkyl group, a saturated or unsaturated $C_{5-12}$-cycloalkyl group or an aryl group or form together a divalent alkylene, cycloalkylene or arylene group with 2 to 12 carbon atoms;
X stands for a halogen atom, particularly for Cl or Br.

The term "independently from each other" in this document means, in the context of substituents, moieties, or groups, that identically designated substituents, moieties, or groups can occur simultaneously with a different meaning in the same molecule.

A "$C_{x-y}$-alkyl" group is an alkyl group comprising x to y carbon atoms, i.e. for example an $C_{1-3}$-alkyl group, is an alkyl group comprising 1 to 3 carbon atoms. The alkyl group can be linear or branched. For example $-CH(CH_3)-CH_2-CH_3$ is considered to be a $C_4$-alkyl group.

A "$C_{x-y}$-cycloalkyl" group is a cycloalkyl group comprising x to y carbon atoms, i.e. for example an $C_{6-8}$-cycloalkyl group, is a cycloalkyl group comprising 6 to 8 carbon atoms. The cycloalkyl group can be linear or branched. For example 4-methylcyclohexyl or 4-isopropylcyclohexyl, respectively, is considered as a $C_7$-cycloalkyl group or $C_9$-cycloalkyl group, respectively.

A "$C_{x-y}$-alkylene" group is an alkylene group comprising x to y carbon atoms, i.e., for example $C_2$-$C_6$ alkylene group is an alkyl group comprising 2 to 6 carbon atoms. The alkylene group can be linear or branched. For example the group $-CH(CH_3)-CH_2-$ is considered as a $C_3$-alkylene group.

In the present document the "water-to-phenol shrinkage" has to be understood as the shrinkage in volume indicated in % by which a given volume of the macroreticular sulfonic acid ion exchange resin being saturated with water is reduced when water is exchanged by phenol and the resin is saturated with phenol. The state of the resin being saturated with water or saturated with phenol, respectively, is achieved, when the volume of the resin being placed in a glass column stays constant upon percolating the resin with water or with phenol, respectively. Since phenol is solid

at room temperature, all operations for measuring the water-to-phenol shrinkage are made at a temperature of 45 °C.

The aromatic compound of formula (I-A) or (I-B) is reacted with an acylating agent of formula (II-A) or (II-B) in the presence of a macroreticular sulfonic acid ion exchange resin offering a water-to-phenol shrinkage between 25 % and 40 %. Those resins are strongly acidic due to their sulfonic acid groups. It has been observed that sulfonic acid ion exchange resins of the gel type are not suitable for the invention. Furthermore, it has been observed that macroreticular sulfonic acid ion exchange resin having a water-to-phenol shrinkage of smaller than 25 % have short service times.

Preferably the water-to-phenol shrinkage is between 28 % and 35 %.

It is preferred that the macroreticular sulfonic acid ion exchange resin is a polystyrene being crosslinked by divinyl benzene in which the crosslinking level is between 5 and 17 %, preferably between 5 and 13 %.

The most preferred macroreticular sulfonic acid ion exchange resin is the resin which is commercially available under the tradename Amberlyst™ 16 WET from Dow Chemicals.

It has been found that this macroreticular sulfonic acid ion exchange resin as described above shows a much more advantageous behaviour than other exchange resins. It particularly shows less deactivation. This leads to the significant advantage that this specific resin type can be used for a much longer period as catalyst for the acylation reaction and, hence, the intervals for re-activation or replacement of the resins are remarkably longer as compared to other resins. In an industrial process this leads to significantly less costs for the reaction resulting in an increase of the space-time-yield (STY).

If a regeneration or re-activation of the resin is necessary, it can be performed at gentle conditions, particularly at temperatures between 15°C and 80°C, preferably between 20° and 50°C. This is very attractive in view of energy costs. The re-activation can be carried out by treatment of the catalyst with strong mineral acids, particularly with aqueous HCl or $HNO_3$ at a temperature of between 15°C and 80 °C, preferably of between 20° and 50°C, followed by washing with water.

The resins of the trademark Amberlyst have been developed and commercialized in the past from Rohm and Haas (now: Dow Chemicals). In general, there are two different types of resins, the one of the gel-type and the other being macroreticular. The gel-type catalysts have no permanent pores, whereas the macroreticular resins have macropores (permanent pores) and are composed of small spherical microgel particles agglomerated together to form clusters. These clusters of microgel particles are glued together at the interfaces. The porosity arises from the void spaces between the clusters. These macroreticular resins swell in the contact with solvents. The swelling properties are an important parameter of the resins. A particularly important and characteristic parameter for the properties of the resins is the water-to-phenol shrinkage.

It has been postulated that the resins used in the method of invention are advantageous because their active sites in the pores of the resins enable reactants to enter the catalyst and products to leave the catalyst after the catalytic reaction took place.

The aromatic compound of formula (I-A) or (I-B) is reacted with an acylating agent of formula (II-A) or (II-B).

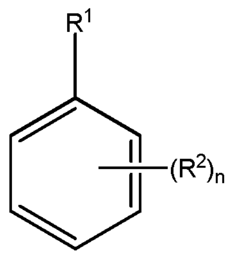

(I-A)

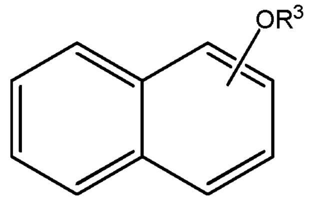

(I-B)

In one embodiment the moiety $R^1$ stands for a $C_{1-4}$-alkyl group. The $C_{1-4}$-alkyl group is methyl, ethyl, n-propyl, isopropyl, sec-butyl or tert.-butyl. Preferably the $C_{1-4}$-alkyl group is methyl or ethyl or isopropyl, most preferably a methyl or ethyl group.

In other embodiment the moiety $R^1$ stands for an $OR^3$ group.

$R^2$ stands for H or for a $C_{1-8}$-alkyl group, particularly for a $C_{1-4}$-alkyl group, or a $C_{6-9}$-cycloalkyl group; particularly for a

$C_{6-8}$-cycloalkyl group.

Suitable examples of aromatic compounds of formula (I-A) or formula (I-B) are:

- 1,2-dimethylbenzene, 1,3-dimethylbenzene, 1,4-dimethylbenzene, 1,2-diethylbenzene, 1,3-diethylbenzene, 1,4-diethylbenzene;
- 1,2,3-trimethylbenzene, 1,2,3-triethylbenzene, 1,3,5,-trimethylbenzene, 1,3,5,-triethylbenzene, 1,2,4-trimethylbenzene, 1,2,4-triethylbenzene;
- methoxybenzene (anisole), ethoxybenzene (phenetole), propoxybenzene, isopropoxybenzene, butoxybenzene, isobutoxybenzene, 2-methylanisole, 3-methylanisole, 2-ethylanisole, 3-ethylanisole, 2-isopropylanisole, 3-isopropylanisole, 2-propylanisole, 3-propylanisole, 2-butylanisole, 3-butylanisole, 1-ethoxy-3-ethylbenzene, 2,3-dimethylanisole, 2,5-dimethylanisole;
- 1,2-dimethoxybenzene (veratrole), 1,3-dimethoxybenzene, 1,4-dimethoxybenzene, 1,2-diethoxybenzene, 1,3-diethoxybenzene, 1,2-dipropoxybenzene, 1,3-dipropoxybenzene;
- 1,2,3-trimethoxybenzene, 1,2,3-triethoxybenzene, 1,3,5-trimethoxybenzene, 1,3,5-triethoxybenzene;
- 1-methoxynaphthalene, 2-methoxynaphthalene.

The aromatic compound formula (I-A) or (I-B) is preferably an aromatic compound of formula (I-A-a).

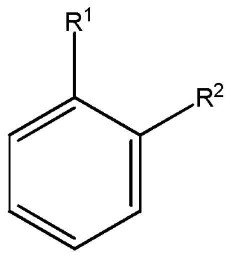

(I-A-a)

For formula (I-A-a) it is, in one embodiment, preferred that $R^1 = R^2$, preferably $R^1 = R^2 = CH_3$.

For formula (I-A-a) it is, in another embodiment, preferred that $R^1 = OCH_3$, preferably $R^1 = OCH_3$ and $R^2 = H$.

Surprisingly, it has been found that aromatic compounds of formula (I-A) in which both $R^1$ and $R^2$ stand for alkyl groups can be acylated by the method of the present invention. Particularly, it was not known yet up to now that for example o-xylene can be acylated to yield to the 1,2-dimethyl-4-acyl-benzene compounds, particularly, 1,2-dimethyl-4-acetyl-benzene (=1-(3,4-dimethylphenyl)ethanone) in the presence of a heterogeneous catalyst. It was furthermore very surprising that the acylation was possible at the particularly low temperatures of between 10°C and 200°C, particularly between 60°C and 120°C, preferably between 80°C and 110°C.

The aromatic compound of formula (I-A) or (I-B) is reacted with an acylating agent of formula (II-A) or (11-B).

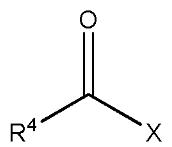

(II-A)

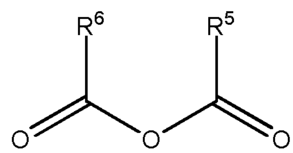

(II-B)

$R^4$ stands for a saturated or unsaturated $C_{1-12}$-alkyl group, a saturated or unsaturated $C_{5-12}$-cycloalkyl group or an aryl group;

$R^5$ and $R^6$ stand independently from each other for a saturated or unsaturated $C_{1-12}$-alkyl group, a saturated or unsaturated $C_{5-12}$-cycloalkyl group or an aryl group or form together a divalent alkylene, cycloalkylene or arylene group with 2 to 12 carbon atoms;

X stands for a halogen atom, particularly for Cl or Br. Preferably X stands for Cl.

The acylating agents are, therefore, either anhydrides of carboxylic acids or acyl halides.

The anhydride may be a symmetrical anhydride ($R^5$=$R^6$) or mixed anhydride (unsymmetrical anhydride) ($R^5$ is different from $R^6$). Preferably the moieties $R^5$ and $R^6$ are identical, i.,e. preferred anhydrides are symmetrical anhydride ($R^5$=$R^6$).

The anhydride is preferably selected from the group consisting of acetic anhydride, propionic anhydride, isobutyric anhydride, benzoic anhydride; phthalic anhydride, hexahydroisobenzofuran-1,3-dione, malonic anhydride, glutaric anhydride, adipic anhydride and pimelic anhydride. Particularly preferred is acetic anhydride.

The acyl halide is preferably selected from the group existing of acetyl chloride, acetyl bromide, propionyl chloride, propionyl bromide, isobutyryl chloride, isobutyryl bromide, pivaloyl chloride, pivaloyl bromide, crotonoyl chloride, crotonoyl bromide, benzoyl chloride, benzoyl bromide, hexahydrobenzoyl chloride and hexahydrobenzoyl bromide.

It is preferred that the acylating agent of formula (II-A) or (II-B) is of formula (II-B), and is particularly acetic acid anhydride.

Compound of formula (I-A) or (I-B) and acylating agent of formula (II-A) or (II-B) are advantageously reacted in the presence of the macroreticular sulfonic acid ion exchange resin in liquid phase, comprising mentioned compound of formula (I-A) or (I-B) and acylating agent of formula (II-A) or (II-B).

Principally, it is possible to add an organic solvent to the reaction mixture. Examples for suitable solvents are aliphatic or aromatic hydrocarbons, halogenated aliphatic or aromatic hydrocarbons, aliphatic, cycloaliphatic or aromatic ethers and polar organic solvent. Organic solvent can also be used as mixtures of solvents.

The amount of solvents is usually selected in a way that the ratio between solvent and aromatic compound of formula (I-A) or (I-B) can be easily varied.

It has been shown that the reaction can be carried out preferably in the absence of any additional organic solvents. In addition to that, one of the starting materials can be used as a solvent. Therefore, it is preferred not to use any additional solvents. As no solvents need to be separated from the acylated aromatic compounds, the process involves less work, less cost, less energy consumption and less waste material treatment.

Hence, it is preferred that the aromatic compound of formula (I-A) or (I-B) is reacted in the absence of any organic solvents with the acylating agent of formula (II-A) or (II-B) in the presence of the macroreticular sulfonic acid ion exchange resin.

The aromatic compound of formula (I-A) or (I-B) is reacted with the acylating agent of formula (II-A) or (II-B) in the presence of a macroreticular sulfonic acid ion exchange resin having a water-to-phenol shrinkage as described above and optionally in the presence of an organic solvent, preferably in the absence of any organic solvents.

The amount of compound of formula (I-A) or (I-B) is preferably selected in amount so that the molar ratio of aromatic compound of formula (I-A) or (I-B) to acylating agent of formula (II-A) or (II-B) is between 0.1 and 35, particularly between 0.5 and 30, preferably between 0.6 and 20, more preferably between 1 and 10.

The amount of the macroreticular sulfonic acid ion exchange resin may vary in relative large ranges.

There are principally two methods suited for the reaction.

In the first of these methods the reaction of aromatic compound of formula (I-A) or (I-B) with an acylating agent of formula (II-A) or (II-B) is performed in a discontinuous manner, e.g. in a slurry reactor.

In the discontinuous manner the amount of macroreticular sulfonic acid ion exchange resin to aromatic compound of formula (I-A) or (I-B) can be varied in large ranges, and is chosen particularly adjusted to the starting materials and the batch size. In certain cases this amount varies from 0.01 to 800 % by weight. Particularly an amount is chosen which is preferably between 0.01 and 50 % by weight, preferably between 1 and 20 % by weight.

The sequence of adding starting materials is not critical in the discontinuous manner of reaction. After adding the starting material the reaction occurs preferably under stirring and heating to the desired temperature.

In the second of the above methods the reaction of aromatic compound of formula (I-A) or (I-B) with an acylating agent of formula (II-A) or (II-B) is performed in a continuous manner, e.g. in a plug flow reactor.

In the continuous manner, it is preferred that the Weight Hourly Space Velocity (WHSV) is 0.01 - 10 per hour, preferably 0.02 - 3 per hour, more preferably 0.05 - 3 per hour.

In the continuous manner the reaction occurs preferably in a tubular fixed bed reactor or in a multi tube reactor or in a moving bed reactor or in a fluidized bed reactor or in a plate reactor with a fixed bed of the resin.

The aromatic compound of formula (I-A) or (I-B) and the acylating agent of formula (II-A) or (II-B) can be added separately or as a mixture thereof.

The retention time ($\tau$) of the material on the fixed bed varies typically between 0.5 seconds and 10 hours, particularly between 30 seconds and 3 hours, preferably between 2 minutes and 1 hour.

It is preferred that the reaction of aromatic compound of formula (I-A) or (I-B) with an acylating agent of formula (II-A) or (II-B) is performed at a temperature of between 10°C and 200°C, particularly between 60°C and 120°C, preferably between 80°C and 110°C.

Usually the reaction occurs at ambient pressure, however, also higher or or reduced pressure can be applied. In case of reacting at a temperature above the boiling point of the starting materials or organic solvent the reaction or products formed can occur in a closed reaction vessel at autogenic pressure.

At the end of the reaction the reaction mixture leaves the reactor. If the resin is fixed in the reactor, no further catalyst separation has to be carried out. If the resin is not fixed in the reactor, the catalyst can be easily separated by filtration or decantation from the reaction product.

At the end of the reaction a liquid phase comprising the acylated aromatic compound formed by the described acylating reaction is obtained. Preferably the acylated aromatic compound has the formula (III-A-a) or (III-A-b) or (III-A-c) or (III-B-a) or (III-B-b) or (III-B-c).

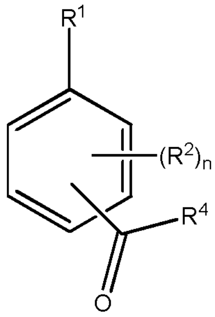
(III-A-a)

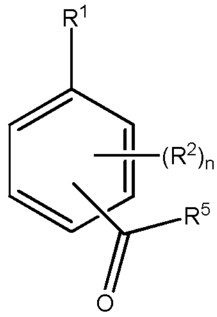
(III-A-b)

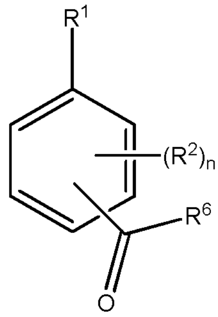
(III-A-c)

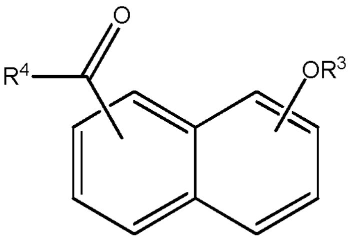
(III-B-a)

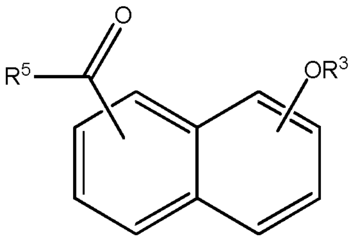
(III-B-b)

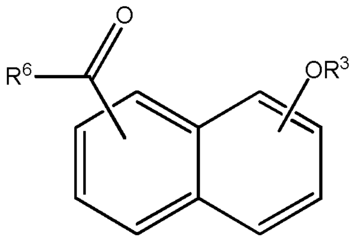
(III-B-c)

The acylated aromatic compound can be isolated and purified further by distillation or re-crystallisation from a suitable solvent.

It has been observed that the acylation reaction is predominately taking place in the para-position of alkyl or alkoxy substituents being present in the aromatic compound which is to be acylated. In cases where the acylation primarily does not take place in the para-position the compound formed can be easily isomerized to the compound with the acyl group in the para-position. The intermediate isomerization can be performed particularly by the method disclosed by E. Fromentin, J.-M. Coustard and M. Guisnet in J. Catal. 190, 433-438 (2000).

It, furthermore, has been observed that multiple acylation can be avoided to a large extent by the parameters and stoichiometry of the reaction. Reaction products formed by an acylation reaction which took place at a different position at the aromatic ring or multiple acylations are typically unfavourable as they are the undesired reaction products and, therefore, decrease the conversion to the desired compound and increase work for their separation from the undesired products.

It is preferred that the acyl group is in the para-position to the group $R^1$.

The acylated aromatic compound formed by the described acylating reaction is preferably selected from the group consisting of 1-(4-methoxyphenyl)-ethanone, 1-(6-methoxynaphthalen-2-yl)ethanone, 1-(4-methoxyphenyl)propan-1-one, 1-(4-methoxyphenyl)-2-methylpropan-1-one; 1-(6-methoxynaphthalen-2-yl)ethanone, 1-(6-methoxynaphthalen-2-yl)propan-1-one, 1-(6-methoxy-naphthalen-2-yl)-2-methylpropan-1-one; 1-(p-tolyl)ethanone, 1-(p-tolyl)propan-1-one, 2-methyl-1-(p-tolyl)propan-1-one; 1-(3,4-dimethylphenyl)ethanone, 1-(3,4-diethylphenyl)ethanone, 1-(3,4-dimethylphenyl)propan-1-one, 1-(3,4-dimethylphenyl)-2-methylpropan-1-one, 1-mesitylethanone and 1-(2,3,4-trimethylphenyl)ethanone.

Particularly important is the acylation of 2-methoxynaphthalene leading to 1-(6-methoxynaphthalen-2-yl)ethanone which is an important starting material for the synthesis of S-Naproxen (=(S)-3-(6-methoxynaphthalen-2-yl)but-1-en-2-ol or (+)-(S)-2-(6-methoxynaphthalen-2-yl)propanoic acid) which is an important pain killer and anti-inflammatory drug.

It has been found that the present method of acylating an aromatic compound of formula (I-A) or (I-B) shows good conversion and excellently high selectivity.

The term "conversion" is defined in the present document in the following manner:

$$\text{conversion} = \left(1\text{-}\left(\frac{a_{end}}{a_{start}}\right)\right)*100$$

wherein $a_{end}$ stands for the number of moles of the aromatic compound of formula (I-A) or (I-B) being present in the reaction mixture after the reaction and $a_{start}$ stands for the number of moles of the aromatic compound of formula (I-A) or (I-B) being present at the begin of the reaction.

The term "selectivity" is defined in the present document in the following manner:

$$\text{selectivity} = \left(1\text{-}\left(\frac{b_{acyl}}{(a_{start} - a_{end})}\right)\right)*100$$

wherein $a_{start}$ stands for the number of moles of the aromatic compound of formula (I-A) or (I-B) being present at the begin of the reaction and $b_{acyl}$ stands for the number of moles of the aromatic compound of formula (I-A) or (I-B) being acylated by the reaction.

The term "yield" is defined in the present document in the following manner:

$$\text{yield} = (\text{conversion} * \text{selectivity})/100$$

**Examples**

The present invention is further illustrated, however, not limited thereto, by the following experiments.

Resins

Amberlyst™ 16 WET is a macroreticular sulfonic acid ion exchange resin having a water-to-phenol shrinkage of 32 % and a crosslinking level of 12 %.

Amberlyst™ 15 WET is a macroreticular sulfonic acid ion exchange resin having a water-to-phenol shrinkage of 13 % and a crosslinking level of 20 %.

Amberlyst™ 36 WET is a macroreticular sulfonic acid ion exchange resin having a water-to-phenol shrinkage of 20 % and a crosslinking level of about 18 %. Amberlyst™ 31 WET is a gel-type sulfonic acid exchange resin having a water-to-phenol shrinkage of 47 %.

Example 1: Acylation of anisole in a continuous manner

The resin has been preconditioned during one hour by contacting it with the reaction mixture at room temperature.

13 mL of Amberlyst™ 16 WET (Dow Chemicals) have been added to a vertically oriented tubular fixed bed reactor equipped with a heating mantle. The fixed bed reactor was then heated up to a temperature of 110°C. A mixture of anisole and acetic anhydride in a molar ratio of anisole to acetic anhydride of 0.9 is introduced at the top of the reactor by means of a membrane pump to the reactor at a Weight Hourly Space Velocity (WHSV) of 0.2 per hour. The reaction mixture is removed continuously at the bottom of the reactor. Conversion and selectivity as defined above are determined by analysing samples by gas chromatography at reaction service times indicated in table 1.

Table 1. Conversion, selectivity and yield of the acylation of anisole.

| Service time [h] | Conversion [%] | Selectivity [%] | Yield [%] |
|---|---|---|---|
| 2 | 22 | 95 | 20.9 |
| 100 | 15 | 95 | 14.3 |
| 220 | 14 | 95 | 13.3 |

The results of example 1 show that the conversion and selectivity remain also at long service times at a high level. The exchange resin is, hence, not deactivated rapidly.

Re-activation of catalyst

The catalyst Amberlyst™ 16 WET was after a service time of 220 hours in example 1 re-activated in the following manner:

5 g of the catalyst to be re-activated have been treated at room temperature (25°C) during 30 minutes by 200 mL aqueous 3M $HNO_3$. The resin has then been washed by distilled water to remove any excess of $HNO_3$ from the resin surface. Afterwards, the catalyst can be used again and shows essentially the same level of conversion and selectivity fresh Amberlyst™ 16 WET does.

Example 2: Acylation of anisole in a continuous manner

The resin has been preconditioned during one hour by contacting it with the reaction mixture at room temperature.

400 mL of Amberlyst™ 16 WET (Dow Chemicals) have been added to a vertically oriented tubular fixed bed reactor equipped with a heating mantle. The fixed bed reactor was then heated up to a temperature of 110°C. A mixture of anisole and acetic anhydride in a molar ratio of anisole to acetic anhydride of 0.9 is introduced at the top of the reactor by means of a membrane pump to the reactor at a Weight Hourly Space Velocity (WHSV) of 0.02 per hour. The reaction mixture is removed continuously at the bottom of the reactor. Conversion and selectivity as defined above are determined by analysing samples by gas chromatography at reaction service times indicated in table 2.

Table 2. Conversion, selectivity and yield of the acylation of anisole.

| Service time [h] | Conversion [%] | Selectivity [%] | Yield [%] |
|---|---|---|---|
| 55 | 23 | 98 | 22.5 |
| 457 | 22 | 94 | 20.7 |
| 652 | 22 | 94 | 20.7 |
| 1000 | 22 | 94 | 20.7 |

The results of example 2 show that the conversion and selectivity remain also at long service times at almost the same high level. The exchange resin is, hence, not deactivated rapidly.

Example 3: Acylation of anisole in a continuous manner (comparison)

The resin has been preconditioned during one hour by contacting it with the reaction mixture at room temperature.

3 mL of Amberlyst™ 31 WET (Dow Chemicals), which is a gel-type and not a macroreticular (as in example 1 and 2) sulfonic acid exchange resin, have been added to a vertically oriented tubular fixed bed reactor equipped with a heating mantle. The fixed bed reactor was then heated up to a temperature of 110°C. A mixture of anisole and acetic anhydride in a molar ratio of anisole to acetic anhydride of 0.9 is introduced at the top of the reactor by means of a membrane pump to the reactor at a Weight Hourly Space Velocity (WHSV) of 5 per hour. The reaction mixture is removed continuously at the bottom of the reactor. Conversion and selectivity as defined above are determined by analysing samples by gas chromatography at reaction service times indicated in table 3.

Table 3. Conversion, selectivity and yield of the acylation of anisole using a gel-type exchange resin.

| Service time [h] | Conversion [%] | Selectivity [%] | Yield [%] |
|---|---|---|---|
| 2 | 11 | 96 | 10.6 |
| 4 | 6 | 100 | 6 |
| 6 | 1 | 100 | 1 |

The results of example 3 show that the conversion at the beginning is very low and drops rapidly to a value of almost no conversion.

The gel-type exchange resin is, hence, rapidly deactivated.

Example 4: Acylation of anisole in a continuous manner (comparison)

The resin has been preconditioned during one hour by contacting it with the reaction mixture at room temperature.

3 g of Amberlyst™ 15 WET (Dow Chemicals) have been added to a vertically oriented tubular fixed bed reactor equipped with a heating mantle. The fixed bed reactor was then heated up to a temperature of 110°C. A mixture of anisole and acetic anhydride in a molar ratio of anisole to acetic anhydride of 1.2 is introduced at the top of the reactor by means of a membrane pump to the reactor at a Weight Hourly Space Velocity (WHSV) of 0.2 per hour. The reaction mixture is removed continuously at the bottom of the reactor. Conversion and selectivity as defined above are determined by analysing samples by gas chromatography at reaction running times indicated in table 4.

Table 4. Conversion, selectivity and yield of the acylation of anisole using Amberlyst 15™ WET exchange resin.

| Running time [min] | Conversion [%] | Selectivity [%] | Yield [%] |
|---|---|---|---|
| 45 (=0.75 h) | 62 | 98 | 60.8 |
| 180 (= 3 h) | 19 | 98 | 18.6 |
| 300 (=5 h) | 11 | 98 | 10.8 |

The results of example 4 show in comparison with examples 1 and 2, that the conversion drops significantly faster when using Amberlyst™ 15 WET than Amberlyst™ 16 WET.

Example 5: Acylation of 2-methoxynaphthalene: batch (discontinuous) manner

In a 100 mL flask being equipped with a water separator 1 g (6.3 mmol) of 2-methoxynaphthalene, 19 g (186 mmol) acetic anhydride and 6 g of Amberlyst™ 16 WET (Dow Chemicals) have been added. The mixture was then heated up under stirring to a temperature of 90°C. After 66 hours reaction time and the reaction mixture was analysed by gas chromatography. Conversion of 2-methoxynaphthalene was 90.6 % and the selectivity to 2-acetyl-6-methoxynaphthalene was 59.4% (yield = 53.8%).

Example 6: Acylation of anisole: batch (discontinuous) manner

In a 250 mL flask being equipped with a water separator 14.3 g (132 mmol) of anisole, 13.5 g (132 mmol) acetic anhydride and 0.72 g of Amberlyst™ 16 WET (Dow Chemicals) have been added. The mixture was then heated up under stirring to a temperature of 60°C. After 6 hours reaction time and the reaction mixture was analysed by gas chromatography. Conversion of anisole was 92.6 % and the selectivity to 1-(4-methoxyphenyl)ethanone was 94.5 % (yield = 87.5%).

Example 7: Acylation of anisole: batch (discontinuous) manner (comparison)

In a 250 mL flask being equipped with a water separator 10 g (92 mmol) of anisole and 0.5 g of the catalysts mentioned in table 5 have been added and heated to 60°C. During 4 hours 9.4 g (92 mmol) acetic anhydride have been added to the reaction mixture by a pump. After the addition of acetic anhydride was finished, the reaction mixture was stirred for an additional 2 hours. Thereafter the reaction mixture was analysed by gas chromatography.

Table 5. Conversion, selectivity and yield of the different catalyst resins.

| Catalyst | Conversion [%] | Selectivity [%] | Yield [%] |
|---|---|---|---|
| Amberlyst™ 15 WET | 55 | 97 | 53.5 |
| Amberlyst™ 31 WET | 26 | 100 | 26 |
| Amberlyst™ 36 WET | 36 | 100 | 36 |

The results of table 5 show that the resins Amberlyst™ 15, Amberlyst™ 31 and Amberlyst™ 36 have a much lower conversion and yield than the Amberlyst™ 16 as used in example 6.

Example 8: Acylation of 1,2-dimethylbenzene: batch (discontinuous) manner

In a 100 mL flask being equipped with a water separator 10 g (94 mmol) of 1,2-dimethylbenzene (o-xylene), 20.9 g (102 mmol) acetic anhydride and 6.2 g of Amberlyst™ 16 WET (Dow Chemicals) have been added. The mixture was then heated up under stirring to a temperature of 110°C. After 24 hours reaction time the reaction mixture was analysed by gas chromatography. Conversion of 1,2-dimethylbenzene was 66.3 % and the selectivity to 3,4-dimethylacetophenone was 9.8% (yield = 6.5%).

The selectivity may be further increased by using a higher amount of catalyst and the optimization of the reaction parameters.

## Claims

1. Method of acylating an aromatic compound of formula (I-A) or (I-B) **characterized in that** the aromatic compound of formula (I-A) or (I-B) is reacted with an acylating agent of formula (II-A) or (II-B) in the presence of a macroreticular sulfonic acid ion exchange resin having a water-to-phenol shrinkage between 25 % and 40 %

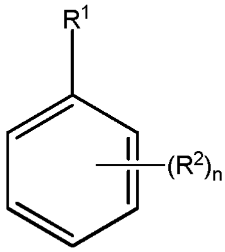

(I-A)

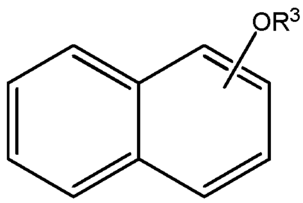

(I-B)

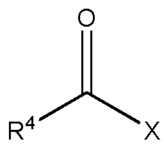

(II-A)

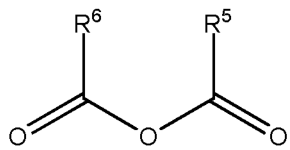

(II-B)

wherein n = 0 to 3, preferably n = 0 or 1;
$R^1$ stands for a $C_{1-4}$-alkyl group or an $OR^3$ group;
$R^2$ stands for H or for a $C_{1-8}$-alkyl group or a $C_{6-9}$-cycloalkyl group;
$R^3$ stands for $C_{1-8}$-alkyl group;
$R^4$ stands for a saturated or unsaturated $C_{1-12}$-alkyl group, a saturated or unsaturated $C_{5-12}$-cycloalkyl group or an aryl group;
$R^5$ and $R^6$ stand independently from each other for a saturated or unsaturated $C_{1-12}$-alkyl group, a saturated or unsaturated $C_{5-12}$-cycloalkyl group or an aryl group or form together a divalent alkylene, cycloalkylene or arylene group with 2 to 12 carbon atoms;
X stands for a halogen atom, particularly for Cl or Br.

2. Method according to claim 1, **characterized in that** the water-to-phenol shrinkage is between 28 % and 35 %.

3. Method according to claim 1 or 2, **characterized in that**, the aromatic compound formula (I-A) or (I-B) is of formula (I-A-a)

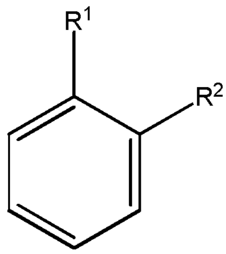

(I-A-a).

4. Method according to claim 3 **characterized in that** $R^1 = R^2$, preferably $R^1 = R^2 = CH_3$

5. Method according to claim 3 **characterized in that** $R^1 = OCH_3$, preferably R'= $OCH_3$ and $R^2$ = H.

6. Method according to anyone of the preceding claims **characterized in that** the acylating agent of formula (II-A) or (II-B) is of formula (II-B), and is particularly acetic acid anhydride.

7. Method according to anyone of the preceding claims **characterized in that** the molar ratio of aromatic compound of formula (I-A) or (I-B) to acylating agent of formula (II-A) or (II-B) is between 0.1 and 35, particularly between 0.5 and 30, preferably between 0.6 and 20, more preferably between 1 and 10.

8. Method according to anyone of the preceding claims **characterized in that** the reaction of aromatic compound of formula (I-A) or (I-B) with an acylating agent of formula (II-A) or (II-B) is performed at a temperature between 10°C and 200°C, particularly between 60°C and 120°C.

9. Method according to anyone of the preceding claims **characterized in that** the reaction of aromatic compound of formula (I-A) or (I-B) with an acylating agent of formula (II-A) or (II-B) is performed in a discontinuous manner.

10. Method according to claim 9, **characterized in that** the amount of macroreticular sulfonic acid ion exchange resin to aromatic compound of formula (I-A) or (I-B) is between 0.01 and 50 % by weight, preferably between 1 and 20 % by weight.

11. Method according to anyone of the preceding claims **characterized in that** the reaction of aromatic compound of formula (I-A) or (I-B) with an acylating agent of formula (II-A) or (II-B) is performed in a continuous manner.

12. Method according to claim 11, **characterized in that** the Weight Hourly Space Velocity (WHSV) is 0.01 - 10 per hour, preferably 0.02 - 3 per hour.

13. Method according to anyone of the preceding claims **characterized in that** the aromatic compound of formula (I-A) or (I-B) is reacted in the absence of any organic solvents with the acylating agent of formula (II-A) or (II-B) in the presence of the macroreticular sulfonic acid ion exchange resin.

14. Method according to anyone of the preceding claims **characterized in that** the macroreticular sulfonic acid ion exchange resin is a macroreticular sulfonic acid ion exchange resin is a polystyrene being crosslinked by divinyl benzene.

15. Method according to claim 14 **characterized in that** the crosslinking level is between 5 and 17 %, preferably between 5 and 13 %.

**Patentansprüche**

1. Verfahren zur Acylierung einer aromatischen Verbindung der Formel (I-A) oder (I-B), **dadurch gekennzeichnet, dass** man die aromatische Verbindung der Formel (I-A) oder (I-B) in Gegenwart eines makroretikulären Sulfonsäure-Ionenaustauscherharzes mit einem Wasser-zu-Phenol-Schrumpf zwischen 25% und 40% mit einem Acylierungsmittel der Formel (II-A) oder (II-B) umsetzt:

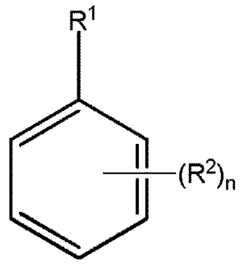

(I-A)

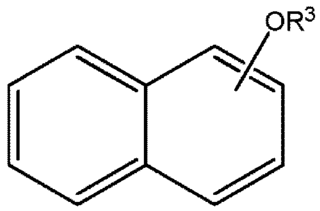

(I-B)

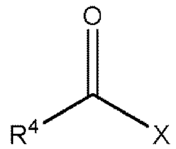

(II-A)

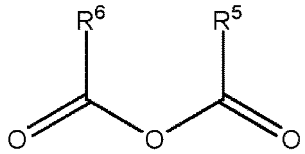

(II-B)

,

wobei n = 0 bis 3, vorzugsweise n = 0 oder 1;
$R^1$ für eine $C_{1-4}$-Alkylgruppe oder eine $OR^3$-Gruppe steht;
$R^2$ für H oder für eine $C_{1-8}$-Alkylgruppe oder eine $C_{6-9}$-Cycloalkylgruppe steht;
$R^3$ für eine $C_{1-8}$-Alkylgruppe steht;
$R^4$ für eine gesättigte oder ungesättigte $C_{1-12}$-Alkylgruppe, eine gesättigte oder ungesättigte $C_{5-12}$-Cycloalkylgruppe oder eine Arylgruppe steht; $R^5$ und $R^6$ unabhängig voneinander für eine gesättigte oder ungesättigte $C_{1-12}$-Alkylgruppe, eine gesättigte oder ungesättigte $C_{5-12}$-Cycloalkylgruppe oder eine Arylgruppe stehen oder zusammen eine zweiwertige Alkylen-, Cycloalkylen- oder Arylengruppe mit 2 bis 12 Kohlenstoffatomen bilden;
X für ein Halogenatom, insbesondere für Cl oder Br, steht.

2. Verfahren nach Anspruch 1, **dadurch gekennzeichnet, dass** der Wasser-zu-Phenol-Schrumpf zwischen 28% und 35% liegt.

3. Verfahren nach Anspruch 1 oder 2, **dadurch gekennzeichnet, dass** die aromatische Verbindung der Formel (I-A) oder (I-B) die Formel (I-A-a) aufweist:

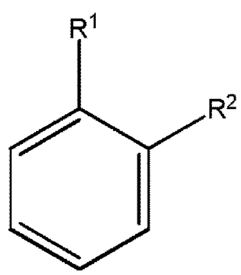

(I-A-a).

4. Verfahren nach Anspruch 3, **dadurch gekennzeichnet, dass** $R^1 = R^2$, vorzugsweise $R^1 = R^2 = CH_3$.

5. Verfahren nach Anspruch 3, **dadurch gekennzeichnet, dass** $R^1 = OCH_3$, vorzugsweise $R^1 = OCH_3$ und $R^2 = H$.

6. Verfahren nach einem der vorhergehenden Ansprüche, **dadurch gekennzeichnet, dass** das Acylierungsmittel der Formel (II-A) oder (II-B) die Formel (II-B) aufweist und insbesondere Essigsäureanhydrid ist.

7. Verfahren nach einem der vorhergehenden Ansprüche, **dadurch gekennzeichnet, dass** das Molverhältnis von aromatischer Verbindung der Formel (I-A) oder (I-B) zu Acylierungsmittel der Formel (II-A) oder (II-B) zwischen 0,1 und 35, insbesondere zwischen 0,5 und 30, vorzugsweise zwischen 0,6 und 20, weiter bevorzugt zwischen 1 und 10, liegt.

8. Verfahren nach einem der vorhergehenden Ansprüche, **dadurch gekennzeichnet, dass** man die Umsetzung der aromatischen Verbindung der Formel (I-A) oder (I-B) mit einem Acylierungsmittel der Formel (II-A) oder (II-B) bei einer Temperatur zwischen 10°C und 200°C, insbesondere zwischen 60°C und 120°C, durchführt.

9. Verfahren nach einem der vorhergehenden Ansprüche, **dadurch gekennzeichnet, dass** man die Umsetzung der aromatischen Verbindung der Formel (I-A) oder (I-B) mit einem Acylierungsmittel der Formel (II-A) oder (II-B) diskontinuierlich durchführt.

10. Verfahren nach Anspruch 9, **dadurch gekennzeichnet, dass** die Menge von makroretikulärem Sulfonsäure-Ionenaustauscherharz zu aromatischer Verbindung der Formel (I-A) oder (I-B) zwischen 0,01 und 50 Gew.-%, vorzugsweise zwischen 1 und 20 Gew.-%, liegt.

11. Verfahren nach einem der vorhergehenden Ansprüche, **dadurch gekennzeichnet, dass** man die Umsetzung der aromatischen Verbindung der Formel (I-A) oder (I-B) mit einem Acylierungsmittel der Formel (II-A) oder (II-B) kontinuierlich durchführt.

12. Verfahren nach Anspruch 11, **dadurch gekennzeichnet, dass** die Katalysatorbelastung (Weight Hourly Space Velocity, WHSV) 0,01-10 pro Stunde, vorzugsweise 0,02-3 pro Stunde, beträgt.

13. Verfahren nach einem der vorhergehenden Ansprüche, **dadurch gekennzeichnet, dass** man die aromatische Verbindung der Formel (I-A) oder (I-B) in Abwesenheit jeglicher organischer Lösungsmittel in Gegenwart des makroretikulären Sulfonsäure-Ionenaustauscherharzes mit dem Acylierungsmittel der Formel (II-A) oder (II-B) umsetzt.

14. Verfahren nach einem der vorhergehenden Ansprüche, **dadurch gekennzeichnet, dass** es sich bei dem makroretikulären Sulfonsäure-Ionenaustauscherharz um ein makroretikuläres Sulfonsäure-Ionenaustauscherharz handelt, bei dem es sich um ein durch Divinylbenzol vernetztes Polystyrol handelt.

15. Verfahren nach Anspruch 14, **dadurch gekennzeichnet, dass** der Vernetzungsgrad zwischen 5 und 17%, vorzugsweise zwischen 5 und 13%, liegt.

## Revendications

1. Procédé d'acylation d'un composé aromatique de formule (I-A) ou (I-B) **caractérisé en ce que** le composé aromatique de formule (I-A) ou (I-B) réagit avec un agent d'acylation de formule (II-A) ou (II-B) en présence d'une résine d'échange d'ions sulfonique macroréticulaire ayant un retrait eau-phénol compris entre 25 % et 40 %

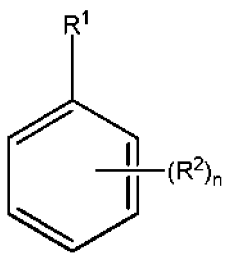

(I-A)

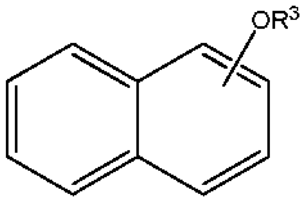

(I-B)

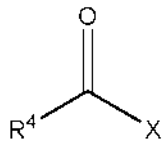

(II-A)

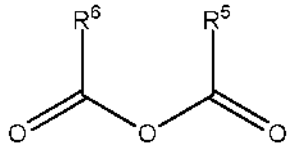

(II-B)

où n = 0 à 3, de préférence n = 0 ou 1 ;
$R^1$ désigne un groupe alkyle en $C_{1-4}$ ou un groupe $OR^3$ ;
$R^2$ désigne H ou un groupe alkyle en $C_{1-8}$ ou un groupe cycloalkyle en $C_{6-9}$ ;
$R^3$ désigne un groupe alkyle en $C_{1-8}$ ;
$R^4$ désigne un groupe alkyle en $C_{1-12}$ saturé ou insaturé, un groupe cycloalkyle en $C_{5-12}$ saturé ou insaturé ou un groupe aryle ;
$R^5$ et $R^6$ désignent indépendamment l'un de l'autre un groupe alkyle en $C_{1-12}$ saturé ou insaturé saturé ou insaturé, un groupe cycloalkyle en $C_{5-12}$ saturé ou insaturé ou un groupe aryle ou forment conjointement un groupe alkylène, cycloalkylène ou arylène divalent avec 2 à 12 atomes de carbone ;
X désigne un atome d'halogène, en particulier Cl ou Br.

**2.** Procédé selon la revendication 1, **caractérisé en ce que** le retrait eau-phénol est compris entre 28 % et 35 %.

**3.** Procédé selon la revendication 1 ou 2, **caractérisé en ce que** le composé aromatique de formule (I-A) ou (I-B) est de formule (I-A-a)

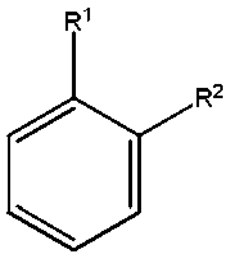

(I-A-a).

**4.** Procédé selon la revendication 3 **caractérisé en ce que** $R^1 = R^2$, de préférence $R^1 = R^2 = CH_3$.

**5.** Procédé selon la revendication 3 **caractérisé en ce que** $R^1 = OCH_3$, de préférence $R^1 = OCH_3$ et $R^2$ = H.

**6.** Procédé selon l'une quelconque des revendications précédentes **caractérisé en ce que** l'agent d'acylation de formule (II-A) ou (II-B) est de formule (II-B), et est, en particulier, l'anhydride d'acide acétique.

**7.** Procédé selon l'une quelconque des revendications précédentes **caractérisé en ce que** le rapport molaire du composé aromatique de formule (I-A) ou (I-B) à l'agent d'acylation de formule (II-A) ou (II-B) est compris entre 0,1 et 35, en particulier entre 0,5 et 30, de préférence entre 0,6 et 20, plus préférablement entre 1 et 10.

**8.** Procédé selon l'une quelconque des revendications précédentes **caractérisé en ce que** la réaction du composé aromatique de formule (I-A) ou (I-B) avec un agent d'acylation de formule (II-A) ou (II-B) est conduite à une température comprise entre 10 °C et 200 °C, en particulier entre 60 °C et 120 °C.

9. Procédé selon l'une quelconque des revendications précédentes **caractérisé en ce que** la réaction du composé aromatique de formule (I-A) ou (I-B) avec un agent d'acylation de formule (II-A) ou (II-B) est conduite de façon discontinue.

10. Procédé selon la revendication 9, **caractérisé en ce que** la quantité de résine d'échange d'ions sulfonique macroréticulaire par rapport au composé aromatique de formule (I-A) ou (I-B) est comprise entre 0,01 et 50 % en poids, de préférence entre 1 et 20 % en poids.

11. Procédé selon l'une quelconque des revendications précédentes **caractérisé en ce que** la réaction du composé aromatique de formule (I-A) ou (I-B) avec un agent d'acylation de formule (II-A) ou (II-B) est conduite de façon continue.

12. Procédé selon la revendication 11, **caractérisé en ce que** la vitesse spatiale horaire en poids (WHSV) est de 0,01 à 10 par heure, de préférence 0,02 à 3 par heure.

13. Procédé selon l'une quelconque des revendications précédentes **caractérisé en ce que** the composé aromatique de formule (I-A) ou (I-B) réagit en l'absence de solvants organiques avec l'agent d'acylation de formule (II-A) ou (II-B) en présence de la résine d'échange d'ions sulfonique macroréticulaire.

14. Procédé selon l'une quelconque des revendications précédentes **caractérisé en ce que** la résine d'échange d'ions sulfonique macroréticulaire est une résine d'échange d'ions sulfonique macroréticulaire qui est un polystyrène étant réticulé par le divinylbenzène.

15. Procédé selon la revendication 14 **caractérisé en ce que** le taux de réticulation est compris entre 5 et 17 %, de préférence entre 5 et 13 %.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 5817878 A

### Non-patent literature cited in the description

- *Org. Proc. Res. Dev.,* 2002, vol. 6, 706-713
- **E. FROMENTIN ; J.-M. COUSTARD ; M. GUISNET.** *J. Catal.,* 2000, vol. 190, 433-438